# EUROPEAN PATENT APPLICATION

(11) **EP 1 270 591 A1**
(43) Date of publication of application: **02.01.2003**
(21) Application number: 02014185.9
(22) Date of filing: 25.06.2002
(51) Int. Cl.: C07K 14/72

(54) **Transgenic cells and a method for detecting disruptors for androgens by using said transgenic cells**

(30) Priority: 25.06.2001 JP 2001190551
(71) Applicant: Okazaki National Research Institutes, Okazaki City, Aichi-Pref. (JP)
(72) Inventor: Nagahama, Yoshitaka, Okazaki city, Aichi Pref. (JP); Ikeuchi, Toshitaka, Okazaki City, Aichi Pref. (JP); Kobayashi, Toru, Okazaki City, Aichi Pref. (JP); Todo, Takashi, Sado-gun, Nigata Pref. (JP)
(74) Representative: Henkel, Feiler, Hänzel

(57) **Abstract**

A transgenic cell is disclosed, where includes a culture cell, and an androgen receptor-expression vector and a foreign reporter gene introduced into said culture cell, said foreign reporter gene having a gene promoter, which is a target of said androgen and an androgen receptor, inserted therein. This transgenic cell is used for detecting a disruptor in a material to be examined through examining the expression of the foreign reporter gene when being brought into contact with the material.

## Description

### Background of the Invention

### (1) Field of the Invention

The present invention relates to transgenic cells and a method for detecting disruptors by using said transgenic cells.

More specifically, the invention relates to transgenic cells utilizing foreign reporter genes, and a method for detecting disruptors by using said transgenic cells.

### (2) Related Art Statement

The hormones are substances which are produced and secreted from specific cells (mainly endocrine cells, and a part of nerve cells) in response to information from the interior and the exterior of the living bodies and which transmit such information to other cells via body fluids. The hormones are broadly classified into three main kinds : peptide hormones such as insulin and glucagon, steroid hormones such as androgen and estrogen, and amine hormones such as adrenalin. Every hormone binds to a hormone receptor inside a target cell, changes its physical property and induces changes in reaction inside the cell. The amine hormones and the peptide hormones act mainly on cell membranes, and control the metabolism inside cells through functional changes of the cell membranes or adjustment of the production of second messengers, whereas the steroid hormones mainly control the expression of the genetic information through binding to receptors inside the cytoplast or nuclei.

Such hormone information constitutes important factors to ensure normal life of the living things, such as functional expression, homeostasis, etc.

Chemical substances that structurally resemble such hormones sometimes disrupt actions of the intrinsic hormones or make them to abnormally act, thereby influencing human bodies and/or generative functions. The artificial organic compounds structurally resembling the hormones are called environmental hormones.

Different from the hormones which are decomposed in livers and discharged outside soon after they terminate their functions, the environmental hormones are difficult to be decomposed, and are dissolve in oil and hardly discharged from the human bodies.

In addition to the above, the environmental hormones have serious problems in that their extremely small amounts function like the normal hormones, cause abnormality against the generative functions regarding the species survival, extreme influences specially upon specific time periods such as fetal stage and abnormality regarding to the foundation of the life such as abnormality in the immune system or abnormality in the behavior.

Under the circumstances, it is an urgent task to search and grasp how much the atmospheres, waters, soils, etc. are contaminated with the environmental hormones.

In general, an equipment called "gas chromatograph mass spectrometer" is known for the analysis of minute amounts of analytes. According to this analysis system, an extraction treatment is effected as a pretreatment that a sample such as soil or water is chemically treated to remove as much ingredients other than a target one as possible. Thereafter, the sample is passed through the analyzer together with a gas, which is subjected to finer separation and measurement of the target ingredient.

However, the above gas chromatograph mass spectrometer requires the extraction operation to preliminarily remove as much the ingredients other than the target one as possible.

If the ingredient to be measured is in a minute amount, the analysis precision is largely deteriorated unless the extraction operation is skillfully performed. Although the quantitative measurement and detection by the gas chromatograph are excellent in examining known substances of which disrupting actions are known. With respect to unknown substances, it is impossible to detect whether such unknown substances exhibit disrupting functions or not. Further, it is impossible to examine how strong the unknown substances act as compared with internal hormones.

Besides, there are many substances that disturb the endocrine system despite of their structural dissimilarity. Such substances can be detected only by checking their actual influences upon living bodies or examining their binding ability to corresponding receptors. Further, problems are that it is troublesome and takes a long time to confirm the influences upon the living bodies, and only examination of the binding ability cannot clarify whether the substances actually function in cells in an accelerated manner or inhibited manner.

Therefore, a detection method that can detect disturbing substances simply even in minute amounts has been desired.

### Summary of the Invention

It is therefore an object of the present invention to provide a disrupting substance-detecting method which can accurately detect a minute amount of androgen function-disrupting substance.

In order to accomplish the above object, the present inventors bound a group of genes acting upon androgens to foreign reporter genes, and made strenuous investigations of transgenic cells containing said gene group. As a result, the inventors discovered the present invention.

The transgenic cell according to the present invention comprises a culture cell comprising a culture cell, and an androgen receptor-expression vector and a foreign reporter gene introduced in the culture cell, the foreign reporter gene having a promoter for a gene, which is a target of said androgen, inserted.

According to a preferred embodiment of the transgenic cell of the present invention, the foreign reporter gene is at least one gene selected from a luciferase gene, a chloramphenicol acetyl transferase (CAT) gene and a β-galactosidase gene.

According to another preferred embodiment of the transgenic cell of the present invention, the androgen hormone receptor-expression vector of the present invention is an androgen receptor α-expression vector or an androgen receptor β-expression vector.

The present invention also relates to a method of detecting a disruptor. The disruptor-detecting method comprises the steps of contacting the above transgenic cell with a material to be examined, detecting the expression of the foreign reporter gene and thereby detecting a disruptor in a disruptor in the material.

According to a preferred embodiment of the disruptor-detecting method of the present invention, the intensity of the expression of the foreign reporter gene is measured based on the intensity of light emission of luciferase.

These and other objects, features and advantages of the invention will be appreciated when taken in conjunction with the attached drawings, with the understanding that some modifications, variations and changes could be made by the skilled in the art to which the invention pertains.

### Brief Description of the Invention

For a better understanding of the invention, reference is made to the attached drawing, wherein:

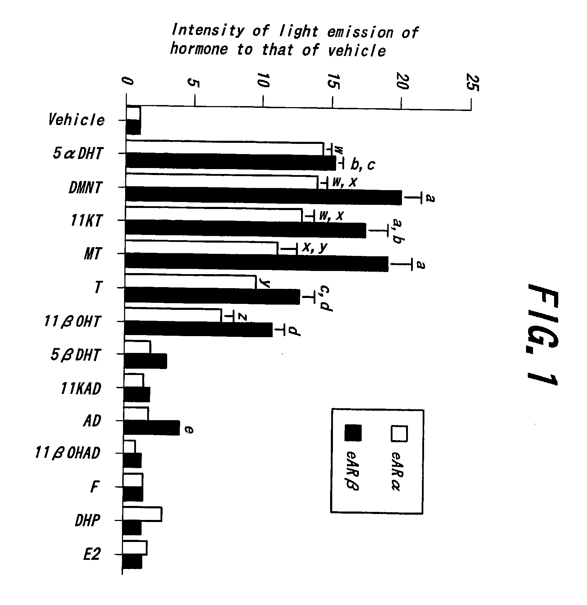

Fig. 1 is a graph showing detected results of various steroids in one example of the present invention.

### Detailed Description of the Invention

The transgenic cell according to the present invention is obtained by introducing into a culture cell an androgen receptor-expression vector and a foreign reporter gene into which is inserted a promoter for a gene that is a target of the androgen and an androgen receptor.

First, how to construct the androgen receptor expression vector will be explained. For example, the expression vector may be constructed by amplifying cDNA fragments adapted for translating an androgen receptor α or an androgen receptor β by PCR using primers which introduced EcoRI site, and ligated PCR resultant with an expression vector, pcDNA 3.1(+) manufactured by Invitrogen Co., Ltd. at the EcoRI site by using a DNA ligase.

Results similar to those in the present invention can be realized by placing the androgen receptor in a nucleus. However, it is extremely difficult to obtain the similar results by placing the receptor in the nucleus as compared with the case where the androgen receptor is synthesized by the expression vector within the cell. From this point of view, the androgen receptor expression vector is constructed and introduced into the cell.

The androgen receptor expression vector is introduced to express the androgen receptor in the nucleus of the cell. The androgen receptor binds to the disruptor to form an androgen receptor-disruptor complex. Therefore, the disruptor acts in the same manner as the androgen or exerts an inhibiting function against the androgen. By utilizing such a function and mechanism, the transgenic cell according to the present invention can be used to detect the disruptor.

According to another aspect of the present invention, a plurality of androgen receptor expression vectors such as the androgen receptor α expression vectors or the androgen receptor β expression vectors may be used as the above androgen receptor expression vector. By using the plural androgen receptor expression vectors, the disruptors can be captured at a higher accuracy. That is, even the disruptor that fails to bind to the receptor α may bind to other receptor(s) the androgen receptor β, so that the disruptors may be detected without failure.

As the androgen receptor expression vector, one originated from a verbrate may be used. For example, an androgen receptor originating from a human being, a mouth, an eel or the like may be used. From the standpoint of view that two kinds of androgen receptor expression vectors can be obtained, the androgen receptors originating from the eel may be preferably recited.

Next, how to construct the foreign reporter gene to which the promoter for the gene that is to be targeted by the androgen is inserted will be explained.

First, a promoter region for a gene which is to be targeted by an androgen receptor (hereinafter referred to as "target gene") is obtained. Since there are plural target genes for the androgen receptor, any of the target sequences may be used. As the promoter region for the target gene, a vector such as pMSG manufactured by Pharmacia Co., Ltd. may be utilized. Since this vector contains a promoter called LTR of a virus MMTV, this region only may be cut out with two kinds of restriction enzymes : Nhe I and Hind III.

Particularly, MMTV-LTR may be preferably used, because it has a wide applicability.

In the present invention, needless to say, various promoters can be used. However, a promoter of a certain target promoter is expressed in muscle only, and a transcription factor specific to the muscle needs to be coexistent. Therefore, if such a promoter is used, various transcription factors or a culture cell system originating from the muscle is required in this case.

As mentioned above, the promoter region can be obtained. The promoter region of the target gene is bound to the foreign reporter gene according to an ordinary method. For example, cohesive ends are formed for the promoter region and the foreign reporter gene by using a restriction enzyme, and then bound to each other with a DNA ligase or the like. For example, it may be that a promoter called "MMTV-LTR" is cut out from a pMSG vector in Pharmacia Co., Ltd., and their ends are made blunt with Klenow fragment of DNA polymerase after using two restriction enzymes : Nhe I and Hind III, and can be bound, with use of a DNA ligase, to Sma I-digested ends of a luciferase expression vector commercially available from Promega Co., Ltd. which produce blunt ends of a protein translation portion of a luciferase (corresponding to the foreign reporter gene).

The androgen target gene-bonded foreign reporter gene can be constructed in this manner.

The foreign reporter gene is not particularly limited, but such a foreign reporter gene of which mRNA or its expressed protein is easily confirmed is preferred.

From the standpoint that speedy confirmation can be made at high sensitivity, luciferase, chloramphenicol acetyl transpherase (CAT) and β-galactosidase may be recited as the foreign reporter gene.

The transgenic cell according to the present invention is obtained by introducing the androgen receptor expression vector and the foreign reporter gene into which the androgen target gene promoter is inserted into the culture cell. In order to enable the foreign reporter gene to effectively reach the translation, another vector may be introduced into the transgenic cell. In the present system, it is possible that additional genes, which are other reporters or transcription factors and so on, can be contransfected trangently. In this case, internal control vector such as pRL-TK, which contains the seapansy (*Renilla reniformis*) luciferase gene with the herpes simplex virus thymidine kinase promoter in order to normalize for variation in transfection effeciency, may be used.

An ordinary method can be used to introduce the foreign reporter gene and the vector into the culture cell. As the gene-introducing method, a phage transduction method, a plasmid gene introduction method, a microinjection method, an electroporation method and a calcium phosphate method may be recited, for example.

Whether the foreign reporter gene and so on are introduced into the culture cell or not can be confirmed, while two kinds of samples : androgen and a solvent into which the androgen is dissolved are. That is, if they are not introduced into the cell, light emission with luciferase is detected in the androgen-added group at an equivalent level to that in the solvent-added group. Thus, the introduction of the foreign reporter gene and the vector into the cell can be confirmed.

The foreign reporter gene and the vector are introduced into the culture cell. If the vector is taken into the cell, it moves into the nucleus. Although it does not selectively move into the nucleus, it can certainly move into the nucleus.

Next, the method for detecting the disruptor according to the present invention will be explained.

According to the disruptor-detecting method of the present invention, the transgenic cell of the invention is contacted with a material to be examined, and the expression of the foreign reporter gene is examine to detect the disruptor.

The principle of the disruptor-detecting method according to the present invention will be first explained. This principle is based on the utilization of the above-mentioned transgenic cell. When the disruptor contacts the transgenic cell, it passes a cell membrane, binds to the androgen receptor inside the nucleus and thereby forms an androgen receptor-disruptor composite. It is considered that this composite causes a change in the conformation of the gene, and this composite acts upon a factor necessary for the expression and for the expression of the foreign reporter gene to which the promoter is inserted. Thereby, the foreign reporter gene downstream the promoter is expressed.

Therefore, when the expression of the foreign reporter gene is checked, whether the material to be examined exhibits an androgen function or an androgen-interrupting function or not can be judged.

Regarding the transgenic cell, see the above explanation. As mentioned above, the foreign reporter gene is not particularly limited.

From the standpoint that definite and speedy confirmation of the disruptor can be made at high sensitivity, luciferase gene, chloramphenicol acetyl transpherase (CAT) gene and β-galaxxidase gene may be recited as the foreign reporter gene.

Materials to be examined which are presumed to contain disruptors are not particularly limited. For example, samples taken from the atmosphere, waters such as rivers and sea water, foods, body fluids of animals and plants, soils, etc. may be used as the materials to be examined. In this case, since it is likely that if such water is used as it is, the culture cells are destroyed with bacteria existing therein, the culture cells can used as being coarsely extracted with an organic solvent such as ethanol, for example.

When the luciferase gene is used as the foreign reporter gene, whether the foreign reporter gene is expressed or not can be quantitatively detected based on the intensity of the light emission of the luciferase protein.

When the chloramphenicol acetyl transpherase (CAT) gene is used as the foreign reporter gene, whether the foreign reporter gene is expressed or not can be quantitatively detected by an enzyme immunoassay or based on the amount of radiations of radioactively labeled chloramphenicol into which an acetyl group is introduced.

When the β-galactosidase gene is used as the foreign reporter gene, whether the foreign reporter gene is expressed or not can be quantitatively detected by the enzyme immunoassay or based on the intensity of a color reaction using a chromophoric substrate.

### (Examples)

In the following, the present invention will be explained in more detail, but the invention should not be interpreted as limited to these examples.

### Example 1

As androgen receptor expression vectors, an eel androgen receptor α expression vector and an eel androgen receptor β expression vector were used. The eel androgen receptors can be obtained by isolating mRNAs from an eel testis, constructing cDNA, and amplifying them with α-specific primers and β-specific primers according to the PCR method, respectively.

The luciferase gene was used as a foreign reporter gene. A commercially available vector (Promega Co., Ltd.) was used as a luciferase expression vector. The foreign reporter gene to which a promoter was inserted was prepared by incorporating a steroid-responsive promoter originated from a long terminal repeat of a murine mammary tumor virus of the upstream of luciferase gene.

Into a 293 cell (culture cell) were transfected 50 ng of the androgen reporter expression vector and 250 ng the luciferase reporter vector inside each hole of a commercially available 24-well culture plate. To the culture cell was added a steroid at 100 nM concentration, which was cultured for 48 hours. More specifically, substances (androgen-like substances) having the androgenic function or an inhibiting ability were detected with use of (1) 7α, 17α-dimethyl-19-nor testosterone (DMNT), (2) 17 α methyl testosterone (MT), (3) 11-ketotestosterone (11KT), (4) 5αdihydrotestosterone (5αDHT), (5) testosterone (T), (6) 11β-hydroxytestosterone (11βOHT), (7) androstenedione, (8) 5β-dihydrotestosterone (5βDHT), (9) 11-ketoandrostenedione (11-KAD), (10) 11β-hydroxyandrostenedione (11βOHAD), (11) 11-ketoetiocholanolone (11KE), (12) estradiol-17b (E2), (13) cortisol (F), and (14) 17α, 20β-dihydroxy-4-pregnene-3-one (DHP(17α, 20β-P as various steroids.

Results obtained are shown in Fig. 1. In Fig. 1, abbreviations such as DMNT denote the various steroids as mentioned above. Further, eARα and eARβ denote the groups in which the eel androgen receptor α expression vector and the eel androgen receptor β expression vector are introduced, respectively.

On the other hand, abcde and wxyz in Fig. 1 show no significant differences between the respective groups (P < 0.01). The intensity of light emission of each of the vehicle groups (hormone solvent, ethanol added) is shown. More specifically, intensity of light emission of each hormone vehicle is shown by the number of times with respect to the intensity of the light emission of the vehicle only.

It is seen from these results that a minite amount of each of the various hormones can be detected at a high sensitivity.

The transgenic cell according to the present invention has an advantageous effect that it can sensitively react upon contacting a minute amount of the disruptor.

The disruptor-detecting method according to the present invention has an advantageous effect that a minute amount of the disruptor can be accurately detected.

Further, according to the disruptor-detecting method of the present invention, whether the substance has a disrupting function or not can be detected, irrespective of being known or unknown.

According to the disruptor-detecting method of the present invention, not only whether the substance has the disrupting function or not but also how strong the power of the disruptor is as compared with that of the inherent endohormone can be examined.

## Claims

1. A transgenic cell comprising a culture cell, and an androgen receptor-expression vector and a foreign reporter gene introduced into said culture cell, said foreign reporter gene having a gene promoter, which is a target of said androgen and an androgen receptor, inserted therein.

2. The transgenic cell set forth in claim 1, wherein the foreign reporter gene is at least one gene selected from a luciferase gene, a chloramphenicol acetyl transferase (CAT) gene and a β-galactosidase gene.

3. The transgenic cell set forth in claim 1 or 2, wherein the androgen receptor-expression vector comprises an androgen receptor α-expression vector and/or an androgen receptor β-expression vector.

4. A method of detecting a disruptor, comprising the steps of contacting said transgenic cell set forth in any one of claims 1-3 with a material to be examined, examining the expression of the foreign reporter gene and thereby detecting a disruptor in said material.

5. The disruptor-detecting method set forth in claim 4, wherein intensity of the expression of the foreign reporter gene is measured based on the intensity of light emission of luciferase, when the luciferase gene is used as the foreign reporter gene.

6. The disruptor-detecting method set forth in claim 4, wherein intensity of the expression of the foreign reporter gene is measured by an enzyme immunoassay or based on the amount of radiations of radioactively labeled chloramphenicol into which an acetyl group is introduced, when the chloramphenicol acetyl transpherase (CAT) gene is used as the foreign reporter gene.

7. The disruptor-detecting method set forth in claim 4, wherein intensity of the expression of the foreign reporter gene is measured by an enzyme immunoassay or based by the enzyme immunoassay or based on the intensity of a color reaction using a chromophoric substrate, when the β-galactosidase gene is used as the foreign reporter gene.
